# EUROPÄISCHE PATENTSCHRIFT

(11) **EP 2 242 848 B1**
(45) Veröffentlichungstag und Bekanntmachung des Hinweises auf die Patenterteilung: **13.04.2011**
(21) Anmeldenummer: 08870064.6
(22) Anmeldetag: 08.12.2008
(51) Int. Cl.: C12P 5/02, C12R 1/145, C02F 11/04

(54) **Clostridium sartagoformum zur Erzeugung von Biogas**
Clostridium sartagoformum for the generation of biogas
Clostridium sartagoformum pour la production de biogaz

(30) Priorität: 10.01.2008 DE 102008003805
(43) Veröffentlichungstag der Anmeldung: 27.10.2010
(73) Patentinhaber: Schmack Biogas GmbH, 92421 Schwandorf (DE)
(72) Erfinder: REUTER, Monika, 84561 Mehring (DE); DUCHOW, Vera, 88471 Laupheim (DE); VATER, Daniel, 02953 Bad Muskau (DE)
(74) Vertreter: Graf Glück Habersack Kritzenberger
(86) Internationale Anmeldenummer: PCT/DE2008/075014
(87) Internationale Veröffentlichungsnummer: WO 2009/086810

(56) Entgegenhaltungen:
- WO-A-2006/056819
- WO-A-2007/052306
- CA-A1- 2 258 254
- NIELSEN HENRIK BANGSO ET AL: "Bioaugmentation of a two-stage thermophilic (68 degrees C/55 degrees C) anaerobic digestion concept for improvement of the methane yield from cattle manure" BIOTECHNOLOGY AND BIOENGINEERING, Bd. 97, Nr. 6, August 2007 (2007-08), Seiten 1638-1643, XP002538156 ISSN: 0006-3592
- HU B ET AL: "Changes in microbial community composition following treatment of methanogenic granules with chloroform" ENVIRONMENTAL PROGRESS & SUSTAINABLE ENERGY, [Online] Bd. 28, Nr. 1, 17. Februar 2009 (2009-02-17), Seiten 60-71, XP002538843
- DATABASE WPI Week 200569 Thomson Scientific, London, GB; AN 2005-670421 XP002538396 & JP 2005 270046 A (MIE TLO KK) 6. Oktober 2005 (2005-10-06)
- PARTANSKY A M ET AL: "Anaerobic Bacteria Capable of the Fermentation of Sulfite Waste Liquor." JOURNAL OF BACTERIOLOGY, Bd. 30, Nr. 6, Dezember 1935 (1935-12), Seiten 559-571, XP002538391 ISSN: 0021-9193
- DATABASE BIOSIS [Online] BIOSCIENCES INFORMATION SERVICE, PHILADELPHIA, PA, US; 2001, SIMUNEK J ET AL: "Chitinolytic bacteria of the mammal digestive tract" XP002538395 Database accession no. PREV200100293003 & FOLIA MICROBIOLOGICA, Bd. 46, Nr. 1, 2001, Seiten 76-78, ISSN: 0015-5632
- DATABASE EMBL [Online] 11. Juli 2006 (2006-07-11), "Uncultured bacterium clone aaa64b04 16S ribosomal RNA gene, partial sequence" XP002538394 Database accession no. DQ817608 & RAWLS JOHN F ET AL: "Reciprocal gut microbiota transplants from zebrafish and mice to germ-free recipients reveal host habitat selection" CELL, Bd. 127, Nr. 2, Oktober 2006 (2006-10), Seiten 423-433, ISSN: 0092-8674
- DATABASE WPI Week 198532 Thomson Scientific, London, GB; AN 1985-194233 XP002538158 & JP 60 122096 A (MATSUSHITA ELEC IND CO LTD) 29. Juni 1985 (1985-06-29)
- AHRING B K: "Biomethanation I, Chapter 5: Optimization of anaerobic digestion" [Online] 2003, SPRINGER-VERLAG BERLIN HEIDELBERG , XP002538392 ISSN: 0724-6145 Gefunden im Internet: URL:http://books.google.de/books?id=qHPxuZ e1868C&pg=PR3&dq=biomethanation+I&client=f irefox-a&hl=en> [gefunden am 2009-07-22] Seiten 22-23, Absatz 5.4
- SNEATH P H A ET AL (EDS.): "Bergey's manual of systematic bacteriology, Volume 2, Section 13: Endospore-forming Gram-positive rods and cocci" 1986, WILLIAMS & WILKINS, BALTIMORE, USA , XP002538393 Seite 1154; Tabelle 13.14 Seiten 1187-88, Absatz 63.

## Beschreibung

### Technisches Gebiet

Die Erfindung betrifft ein Verfahren zur Erzeugung von Biogas aus Biomasse unter Verwendung eines Mikroorganismus der Art *Clostridium sartagoformum.*

### Stand der Technik

Biogasanlagen erzeugen Methan durch einen mikrobiellen Abbauprozess von organischen Substanzen. Das Biogas entsteht dabei in einem mehrstufigen Prozess der Vergärung oder Faulung durch die Aktivität von anaeroben Mikroorganismen, d.h. unter Ausschluss von Luft.

Das als Gärsubstrat verwendete organische Material besitzt aus chemischer Sicht einen hochmolekularen Aufbau, der in den einzelnen Verfahrensschritten einer Biogasanlage durch Stoffwechseltätigkeit der Mikroorganismen zu niedermolekularen Bausteinen abgebaut wird. Die bei der Fermentierung des organischen Gärsubstrats aktiven Populationen von Mikroorganismen sind bislang aber nur unzureichend charakterisiert.

Nach heutigem Kenntnisstand können vier nacheinander, aber auch parallel ablaufende und ineinander greifende biochemische Einzelprozesse unterschieden werden, die den Abbau von organischen Gärsubstraten zu den Endprodukten Methan und Kohlendioxid ermöglichen: Hydrolyse, Acidogenese, Acetogenese und Methanogenese.

In der Hydrolyse werden hochmolekulare, oft partikulär vorliegende, organische Verbindungen durch Exoenzyme (z.B. Cellulasen, Amylasen, Proteasen, Lipasen) fermentativer Bakterien in lösliche Spaltprodukte überführt. Dabei werden beispielsweise Fette in Fettsäuren, Kohlenhydrate, wie z.B. Polysaccharide, in Oligo- und Monosaccharide sowie Proteine in Oligopeptide beziehungsweise Aminosäuren zerlegt. Die daneben gebildeten gasförmigen Produkte bestehen überwiegend aus Kohlendioxid.

Fakultativ und obligat anaerob lebende Bakterien, oftmals identisch mit den hydrolysierenden Bakterien, verstoffwechseln in der Acidogenese die Hydrolyseprodukte (z.B. Mono-, Disaccharide, Di-, Oligopeptide, Aminosäuren, Glycerin, langkettige Fettsäuren) intrazellulär zu kurzkettigen Fett- oder Carbonsäuren, wie beispielsweise Butter-, Propion- und Essigsäure, zu kurzkettigen Alkoholen wie zum Beispiel Ethanol und zu den gasförmigen Produkten Wasserstoff und Kohlendioxid.

In der sich anschließenden Acetogenese werden die in der Acidogenese gebildeten kurzkettigen Fett- und Carbonsäuren sowie die kurzkettigen Alkohole von acetogenen Bakterien aufgenommen und nach β-Oxidation als Essigsäure wieder ausgeschieden. Nebenprodukte der Acetogenese sind -CO₂ und molekularer Wasserstoff (H₂).

Die Produkte der Acetogenese wie Essigsäure aber auch andere Substrate wie Methanol und Formiat werden durch Methan-bildende Organismen in der obligat anaerob verlaufenden Methanogenese zu Methan und CO₂ umgesetzt. Das hier entstehende CO₂ und auch das während der übrigen Prozessschritte, wie z.B. der Hydrolyse, gebildete CO₂ kann wiederum durch Mikroorganismen mit dem angefallenen H₂ ebenfalls zu Methan umgesetzt werden.

Die Erhöhung der Ausbeute an Endprodukten aus einer gegebenen Menge an Edukten stellt wie bei jeder chemischen Umsetzung auch im Fall der Herstellung von Biogas ein vordringliches Ziel der Prozessführung dar. Für die Erzeugung von Biogas aus Biomasse bedeutet dies, dass aus einer gegebenen Menge an organischem Gärsubstrat eine möglichst große Menge an Methan gebildet werden soll.

Daneben soll eine möglichst hohe Raumbelastung der Fermenter erreicht werden. Unter der Raumbelastung eines Fermenters versteht man die Menge an dem Fermenter zugeführten Substrat, die in Kilogramm organischer Trockensubstanz pro Kubikmeter Fermentervolumen und pro Tag angegeben wird. Die Menge an erzeugtem Biogas hängt stark von der Raumbelastung des Fermenters ab, wobei mit steigender Raumbelastung eine zunehmend größere Menge an Biogas erzeugt wird. Eine hohe Raumbelastung macht den Prozess der Biogaserzeugung also zunehmend ökonomisch rentabler, führt andererseits aber zu einer zunehmenden Destabilisierung der biologischen Prozesse der Fermentierung.

WO 2006/056819 offenbart ein Verfahren zur Steigerung der Produktion von Biogas aus Biomasse, dadurch gekennzeichnet, dass der Biomasse ein thermophiler, acetogener und Wasserstoff produzierender Mikroorganismus zugegeben wird, beispielsweise ein Mikroorganismus des Genus *Thermotoga, Thermococcus, Pyrococcus, Clostridium, Ruminococcus* oder *Caldicellulosiruptor.*

Nielsen H. B. et al. (2007), Biotechnology and Bioengineering 97 (6), 1638-1643, beschreibt ein weiteres Verfahren zur Steigerung der Produktion von Biogas aus Biomasse, dadurch gekennzeichnet, daß der Biomasse ein Mikroorganismus des Genus *Caldicellusiruptor* oder *Dictyoglomus* zugesetzt wird.

WO 2007/052306 offenbart ein dreistufiges Verfahren zur Herstellung von Biogas aus Biomasse, in welchem in der ersten Stufe (Hydrolyse) ein Mikroorganismus, beispielsweise *Bacillus subtilis* oder *Clostridium propionicum,* zugegeben wird, um die Hydrolyse zu beschleunigen.

WO 2006/119052 offenbart ein Verfahren zur Herstellung von Wasserstoff aus Biomasse durch anaerobe Fermentation, in welchem der Biomasse nach Sterilisation ein Bakterium des Genus *Clostridium* zugesetzt wird.

Es besteht daher weiterhin ein Bedarf an Verfahren zur Erzeugung von Biogas, die eine gegenüber dem Stand der Technik erhöhte Raumbelastung ermöglichen.

### Darstellung der Erfindung

Die Aufgabe der Erfindung besteht darin, ein Verfahren zur Erzeugung von Biogas bereitzustellen, das eine gegenüber dem Stand der Technik erhöhte Raumbelastung des Fermentierungsreaktors ermöglicht.

Diese Aufgabe wird erfindungsgemäß durch das Verfahren zur Erzeugung von Biogas gemäß Anspruch 1 gelöst. Weitere vorteilhafte Details, Aspekte und Ausgestaltungen der vorliegenden Erfindung ergeben sich aus den abhängigen Ansprüchen, der Beschreibung und den Beispielen.

Die vorliegende Erfindung stellt ein Verfahren zur Erzeugung von Biogas aus Biomasse in einem Fermentierungsreaktor zur Verfügung. Der Biomasse wird ein Mikroorganismus der Art *Clostridium sartagoformum* zugesetzt.

Überraschenderweise hat sich gezeigt, dass durch die Zugabe von Mikroorganismen der Art *Clostridium sartagoformum* zum Gärsubstrat sowohl die Raumbelastung des Fermenters gesteigert werden kann als auch die Menge an gebildetem Biogas deutlich erhöht wird. Wie in experimentellen Untersuchungen gezeigt werden konnte, bewirkt die Zugabe eines Mikroorganismus der Art *Clostridium sartagoformum* eine Steigerung der Raumbelastung eines Fermenters um mehr als 50%, ohne dass eine Instabilität des Fermentationsprozesses eintreten würde. Parallel zu der erhöhten Raumbelastung wird die Menge an gebildetem Biogas mehr als verdoppelt. Zudem steigt die spezifische Ausbeute an Biogas an, da deutlich mehr der organischen Trockensubstanz abgebaut wird als bei fehlender Zugabe von Mikroorganismen der Art *Clostridium sartagoformum.* Durch den erhöhten Abbaugrad kann eine deutlich erhöhte spezifische Gasausbeute bei einer verbesserten Substratausnutzung erzielt werden. Durch die Zugabe von Mikroorganismen der Art *Clostridium sartagoformum* kann die Verweilzeit des Gärsubstrats im Fermenter bei konstanter Gasausbeute deutlich verkürzt werden, wodurch die Erhöhung der Raumbelastung möglich wird. Der Einsatz von Mikroorganismen der Art *Clostridium sartagoformum* führt daher zu einer dramatischen Verbesserung von Effizienz und Wirkungsgrad von Biogasanlagen.

Fermentierung im Sinne der vorliegenden Erfindung umfasst sowohl anaerobe, wie auch aerobe Stoffumwandlungen durch die Einwirkung von Mikroorganismen, die zur Erzeugung von Biogas führen. Diese Erläuterung des Begriffs "Fermentierung" ist unter dem Stichwort "Fermentation" im Römpp-Chemielexikon in der 9., erweiterten Auflage, erschienen im Georg Thieme Verlag auf der Seite 1306, angegeben, auf das hiermit vollinhaltlich Bezug genommen wird.

Die spezifische Ausbeute an erzeugtem Biogas berechnet sich aus der Menge an erzeugtem Biogas dividiert durch die Menge an organischer Trockensubstanz. Die Frage, ob die Erzeugung von Biogas in einem bestimmten Fermenter unter bestimmten Bedingungen in einer befriedigenden Güte abläuft, lässt sich nicht alleine anhand der Menge an erzeugtem Biogas beurteilen. Selbstverständlich hängt die Menge an erzeugtem Biogas stark von der Menge an zugeführtem Substrat ab, also von der Menge an organischer Trockensubstanz, die in Kilogramm organischer Trockensubstanz angegeben wird. Treten Instabilitäten des Fermentierungsprozesses auf, so nimmt die spezifische Gasausbeute ab. Bei erfindungsgemäßem Zusatz von Mikroorganismen der Art *Clostridium sartagoformum* kann die Raumbelastung sogar über das übliche Maß hinaus erhöht werden, wodurch eine deutlich gesteigerte Gasmenge erzeugt wird. Durch den erhöhten Abbaugrad kann eine deutlich erhöhte spezifische Gasausbeute bei einer verbesserten Substratausnutzung erzielt werden.

Unter der Bezeichnung "Art von Mikroorganismen" wird im Rahmen der vorliegenden Erfindung die entsprechende grundlegende Kategorie der biologischen Taxonomie verstanden. Arten von Mikroorganismen werden anhand ihrer DNA-Sequenzen identifiziert und unterschieden. Unter eine bestimmte Art fallen dabei nicht nur Mikroorganismen mit einer ganz bestimmten DNA-Sequenz, sondern auch bis zu einem gewissen Umfang deren genetische Varianten. Dem einschlägigen Fachmann ist bekannt, welche Stämme von Mikroorganismen unter den Begriff "Art *Clostridium sartagoformum"* fallen. Aus dem Stand der Technik ist die Isolierung von Mikroorganismen der Art *Clostridium sartagoformum* beispielsweise aus Fäkalien von Säuglingen bekannt (Stark, P.L.; Lee, A.; J. Pediatr. 1982; 100(3), S. 362-365). Im Zusammenhang mit der Herstellung von Biogas durch Fermentation organischer Substrate sind Mikroorganismen der Art *Clostridium sartagoformum* nicht bekannt.

Gemäß der vorliegenden Erfindung wird ein Mikroorganismus der Art *Clostridium sartagoformum* in Form einer Kultur von Mikroorganismen zugesetzt, die überwiegend aus einem Mikroorganismus der Art *Clostridium sartagoformum* besteht. In Gärsubstraten von Biogasanlagen konnten Mikroorganismen der Art *Clostridium sartagoformum* nur in geringsten Spuren von weniger als 10⁻⁴% Anteil an der gesamten Anzahl von anwesenden Mikroorgansimen nachgewiesen werden. Da für die Zugabe der Mikroorganismen die Menge an aus ihrem natürlichen Vorkommen isolierten Mikroorganismen nicht ausreicht, wird üblicherweise eine Vermehrung in Form einer Kultur vorgenommen. In der Praxis zeigt sich, dass die Zugabe der Mikroorganismen zu dem Gärsubstrat eines Fermenters am einfachsten direkt in Form einer Kultur von Mikroorganismen vorgenommen wird.

Die Zugabe der Kultur von *Clostridium sartagoformum* kann in Form einer Kultursuspension, in Form trockener, gefriergetrockneter oder feuchter Zellpellets oder auch in Form von Sporensuspensionen, Sporenpräparaten oder trockener, gefriergetrockneter oder feuchter Sporenpellets vorgenommen werden.

Da die bereits angesprochenen verschiedenen positiven Effekte auf den Gärprozess mit der Mikroorganismenart *Clostridium sartagoformum* verbunden sind, sollte diese Art von Mikroorganismen in der zugegebenen Kultur in einer das natürliche Vorkommen übersteigenden Menge anwesend sein. Selbstverständlich können Mischkulturen in beliebiger Zusammensetzung für die Zugabe verwendet werden. Voraussetzung ist lediglich, dass die Art *Clostridium sartagoformum* in einer gegenüber dem natürlichen Vorkommen angereicherten Menge anwesend ist.

Die Bestimmung der Anteile verschiedener Arten von Mikroorganismen in Mischkulturen stellt für den Fachmann kein Problem dar. So kann beispielsweise mit Hilfe von Fluoreszenz-markierten Oligosonden spezifisch der Anteil von Mikroorganismen der Art *Clostridium sartagoformum* in einer Mischung identifiziert werden. Wie bereits erwähnt, werden Mikroorganismen der Art *Clostridium sartagoformum* in Form von Kulturen von Mikroorganismen dem Gärsubstrat zugesetzt, wobei die Kulturen von Mikroorganismen überwiegend aus Mikroorganismen der Art *Clostridium sartagoformum* bestehen. Wird neben der Bestimmung der Anzahl an Mikroorganismen der Art *Clostridium sartagoformum* auch die Gesamtzahl an Mikroorganismen bestimmt, so kann der Anteil an Mikroorganismen der Art *Clostridium sartagoformum* in der Kultur in Prozent angegeben werden. Mikroorganismen der Art *Clostridium sartagoformum* sind in einer Mischkultur dann die überwiegend anwesende Art von Mikroorganismen, wenn sie den höchsten prozentualen Anteil der verschiedenen in der Mischkultur anwesenden Arten von Mikroorganismen aufweisen.

Gemäß der vorliegenden Erfindung macht der Mikroorganismus *Clostridium sartagoformum* zumindest 1% der Gesamtzahl an in der zu dem Gärsubstrat zugegebenen Kultur vorhandenen Mikroorganismen aus. Gemäß weiteren bevorzugten Ausführungsformen macht der Mikroorganismus *Clostridium sartagoformum* zumindest 10% der Gesamtzahl an in der Kultur vorhandenen Mikroorganismen aus.

Gemäß einer ganz besonders bevorzugten Ausführungsformen wird eine Reinkultur eines Mikroorganismus der Art *Clostridium sartagoformum* zugesetzt. Die Reinkultur eines Mikroorganismus umfasst die Nachkommenschaft einer einzelnen Zelle, die durch einen mehrschrittigen Prozess aus einem Gemisch verschiedener Mikroorganismen isoliert wird. Dieser mehrschrittige Mechanismus beginnt mit der Abtrennung einer einzelnen Zelle aus einer Zellpopulation und erfordert, dass auch die aus der Zelle durch Wachstum und Zellteilung hervorgehende Kolonie von anderen Einzelzellen oder Kolonien getrennt bleibt. Durch eine sorgfältige Abtrennung einer Kolonie, erneutes Suspendieren in Flüssigkeit und wiederholtes Ausstreichen können gezielt Reinkulturen von Mikroorganismen gewonnen werden.

Die Isolierung einer Reinkultur kann jedoch auch in flüssigen Nährmedien erfolgen, sofern der gewünschte Organismus im Ausgangsmaterial zahlenmäßig überwiegt. Durch serienmäßige Verdünnung der Suspension in der Nährlösung lässt es sich schließlich erreichen, dass sich in der letzten Verdünnungsstufe nur noch eine Zelle befindet. Diese Zelle stellt dann die Basis für eine Reinkultur dar. Diese Erläuterung des Begriffs "Reinkultur" und mögliche Verfahren zur Erzeugung dieser Reinkultur sind unter dem Stichwort "Reinkultur" im Werk "Allgemeine Mikrobiologie" von Hans G. Schlegel in der 7. überarbeiteten Auflage von 1992, erschienen im Georg Thieme Verlag auf der Seite 205 aufgeführt, auf das hiermit vollinhaltlich Bezug genommen wird.

Die so erhaltene Reinkultur ist darüber hinaus auch biochemisch durch spezifische Stoffwechselprozesse und -aktivitäten, sowie durch spezielle Wachstumsbedingungen gekennzeichnet. Aufgrund der spezifischen Stoffwechselprozesse und -aktivitäten kann die Zugabe einer Reinkultur eines fermentativen Mikroorganismus in besonderem Maße zu einer verbesserten Kontrolle des komplexen Biogaserzeugungsprozesses beitragen.

Gemäß einer weiteren bevorzugten Ausführungsform der vorliegenden Erfindung wird ein Mikroorganismus der Art *Clostridium sartagoformum* als Bestandteil zumindest einer immobilisierten Kultur von Mikroorganismen zugesetzt. Da für die Zugabe der Mikroorganismen die Menge an aus ihrem natürlichen Vorkommen isolierten Mikroorganismen nicht ausreicht, wird üblicherweise eine Vermehrung in Form einer Kultur vorgenommen. In der Praxis zeigt sich, dass die Zugabe der Mikroorganismen zu dem Gärsubstrat eines Fermenters am einfachsten in Form einer immobilisierten Kultur von Mikroorganismen vorgenommen wird.

Da die bereits angesprochenen verschiedenen positiven Effekte auf den Gärprozess mit der Mikroorganismenart *Clostridium sartagoformum* verbunden sind, sollte diese Art von Mikroorganismen in der zugegebenen immobilisierten Kultur in einer im Vergleich zum natürlichen Vorkommen angereicherten Menge anwesend sein. Selbstverständlich können immobilisierte Mischkulturen in beliebiger Zusammensetzung für die Zugabe verwendet werden. Voraussetzung ist lediglich, dass Mikroorganismen der Art *Clostridium sartagoformum* in einer Menge enthalten sind, die deren natürliches Vorkommen übersteigt.

Gemäß der vorliegenden Erfindung macht der Mikroorganismus der Art *Clostridium sartagoformum* zumindest 1% der Gesamtzahl an in der immobilisierten Kultur vorhandenen Mikroorganismen aus.

Gemäß weiteren bevorzugten Ausführungsformen macht der Mikroorganismus der Art *Clostridium sartagoformum* zumindest 10% der Gesamtzahl an in der immobilisierten Kultur vorhandenen Mikroorganismen aus.

Gemäß einer ganz besonders bevorzugten Ausführungsformen wird zumindest eine immobilisierte Reinkultur eines Mikroorganismus der Art *Clostridium sartagoformum* zugesetzt.

Als Trägermaterialien, auf denen der Mikroorganismus *Clostridium sartagoformum* immobilisiert wird, können natürliche oder synthetische Polymere eingesetzt werden. Bevorzugt werden gelbildende Polymere verwendet. Diese haben den Vorteil, dass Bakterien innerhalb der Gelstruktur aufgenommen bzw. eingelagert werden können. Bevorzugt werden solche Materialien eingesetzt, die sich in Wasser langsam auflösen bzw. abgebaut werden, so dass die Freisetzung des Mikroorganismus *Clostridium sartagoformum* über einen längeren Zeitraum hinweg erfolgt.

Beispiele für geeignete Polymere sind Polyanillin, Polypyrrol, Polyvinylpyrolidon, Polystyrol, Polyvinylchlorid, Polyvinylalkohol, Polyethylen, Polypropylen, Epoxidharze, Polyethylenimine, Polysaccharide wie Agarose, Alginat oder Cellulose, Ethylcellulose, Methylcellulose, Carboxymethylethylcellulose, Celluloseacetate, Alkali-Cellulosesulfat, Copolymere aus Polystyrol und Maleinsäureanhydrid, Copolymere aus Styrol und Methylmethacrylat, Polystyrolsulfonat, Polyacrylate und Polymethacrylate, Polycarbonate, Polyester, Silikone, Cellulosephthalat, Proteine wie Gelatine, Gummi arabicum, Albumin oder Fibrinogen, Gemische aus Gelatine und Wasserglas, Gelatine und Polyphosphat, Gelatine und Copolymere aus Maleinsäureanhydrid und Methylvinylether, Celluloseacetatbutyrat, Chitosan, Polydialkyldimethylammonium-chlorid, Mischungen aus Polyacrylsäuren und Polydiallyldimethylammoniumchlorid sowie deren Gemische. Das Polymermaterial kann auch mit Hilfe üblicher Vernetzer wie Glutaraldehyd, Harnstoff/Formaldehydharzen oder Taninverbindungen vernetzt werden.

Alginate als Immobilisate erweisen sich als besonders vorteilhaft, da sie zum einen keinen negativen Einfluss auf die Aktivität des Mikroorganismus *Clostridium sartagoformum* nehmen und da sie zum anderen durch Mikroorganismen langsam abgebaut werden. Durch den langsamen Abbau der Alginat-Immobilisate werden nach und nach die eingeschlossenen Mikroorganismen der Art *Clostridium sartagoformum* freigesetzt.

Für die Immoblisation werden die Mikroorganismen mit einem Polymergel vermengt und dann in einer geeigneten Härterlösung gehärtet. Dazu werden sie zunächst mit einer Gellösung vermischt und anschließend in eine Härterlösung aus geeigneter Höhe getropft. Die genauen Vorgehensweisen zur Immobilisation sind dem Fachmann bekannt.

Gemäß einer weiteren Ausführungsform wird zeitnah zur Zugabe eines Mikroorganismus der Art *Clostridium sartagoformum* zusätzliche Biomasse in den Fermentierungsreaktor gegeben. Dabei bezieht sich "zeitnah" auf die Zeitspanne, die zwischen einer sich an die Zugabe der Mikroorganismen anschließende Zugabe von neuem Substrat vergeht. Diese Zeitspanne soll möglichst kurz gehalten werden, weshalb der Begriff "zeitnah" auch eine parallele Zugabe des Mikroorganismus *Clostridium sartagoformum* und des neuen Substrats umfasst.

Die genannte Zeitspanne kann sich jedoch auch auf mehrere Stunden bis hin zu einem oder mehreren Tagen ausdehnen. Dabei kann die Raumbelastung im Fermentationsreaktor durch kontinuierliche Zugabe von neuem Substrat kontinuierlich gesteigert oder in etwa konstant gehalten werden, wobei die Fermentierung bei allen Raumbelastungen, bevorzugt bei einer Raumbelastung von ≥ 0.5 kg organischer Trockensubstanz pro m³ und Tag [kg oTS/m³d], weiter bevorzugt bei einer Raumbelastung von ≥4.0 kg oTS/m³d und besonders bevorzugt bei einer Raumbelastung von ≥8.0 kg oTS/m³d durchgeführt werden kann, was im Vergleich zum gegenwärtigen Stand der Technik einer Erhöhung der Raumbelastung um mehr als das Doppelte entspricht.

Gemäß einer weiteren bevorzugten Ausführungsform wird daher die Raumbelastung im Fermentierungsreaktor durch kontinuierliche Zugabe von Biomasse kontinuierlich gesteigert. Besonders bevorzugt erfolgt die Erzeugung von Biogas aus Biomasse bei einer Raumbelastung von ≥ 0,5 kg oTS/m³d, insbesondere bevorzugt ≥ 4,0 kg oTS/m³d und ganz besonders bevorzugt ≥ 8,0 kg oTS/m³d_{.}

Das verwendete Gärsubstrat kann insbesondere auch einen hohen Anteil an festen Bestandteilen aufweisen. Durch die Zugabe eines hydrolytisch aktiven, fermentativen Mikroorganismus der Art *Clostridium sartagoformum* werden diese festen Bestandteile zumindest teilweise verflüssigt. Durch die erzielte Verflüssigung des Gärsubstrats aufgrund der Zugabe des Mikroorganismus *Clostridium sartagoformum* kann einem Eindicken des Fermentermaterials vorgebeugt und gezielt entgegengewirkt werden. Ein weiterer Flüssigkeitseintrag in das Gärsubstrat in Form von Wasser oder Gülle während der Fermentierung kann vermieden werden. Somit besteht ein weiterer Vorteil in der Schonung der Ressource Süßwasser. Ebenfalls von Vorteil ist der so erzielte Erhalt der Rühr- und Pumpfähigkeit des Substrats. Dadurch werden Rührwerke und Pumpen geschont und für den Rührvorgang ist deutlich weniger Energie erforderlich.

Gemäß einer weiteren Ausführungsform erfolgt die Erzeugung von Biogas aus Biomasse unter konstanter Durchmischung des Gärsubstrats. Durch die konstante Durchmischung des Gärsubstrats können die Kulturen von *Clostridium sartagoformum* besser im Gärsubstrat verteilt werden. Außerdem kann das gebildete Biogas besser aus dem Fermentationsprozess abgeführt werden.

Die konstante Durchmischung des Gärsubstrats führt zudem zu einer gleichmäßigen Wärmeverteilung im Fermentationsreaktor. Messungen der Temperatur im Fermentationsreaktor, die in periodischen Abständen, aber auch kontinuierlich durchgeführt wurden, ergaben, dass das Gärsubstrat in einem Temperaturbereich von 20°C bis 80°C, bevorzugt bei etwa 40°C bis 50°C effizient fermentiert wird. Diese Temperaturbereiche werden daher bevorzugt. Neben der Hydrolyse erfolgt insbesondere die letzte Stufe des Fermentationsprozesses, nämlich die Bildung von Methan durch methanogene Mikroorganismen, besonders effizient bei erhöhten Temperaturen.

Bevorzugt erfolgt daher die Erzeugung von Biogas aus Biomasse bei einer Temperatur von 20°C bis 80°C und besonders bevorzugt bei einer Temperatur von 40°C bis 50°C.

Sämtliche Ausführungsformen der vorliegenden Erfindung sind selbstverständlich nicht auf einstufige Verfahren zur Herstellung von Biogas beschränkt. Der Einsatz von Mikroorganismen der Art *Clostridium sartagoformum* kann auch in zwei- oder mehrstufigen Verfahren erfolgen.

Gemäß einer weiteren Ausführungsform werden Gärsubstrat und ein Mikroorganismus der Art *Clostridium sartagoformum* kontinuierlich zugegeben. Der kontinuierliche Betrieb eines Fermentationsreaktors soll bei einer stabilen mikrobiellen Biozönose zu einer kontinuierlichen Produktion von Biogas führen, wobei das Aussetzen der Substratzugabe zur Fermentation infolge einer Prozessstörung vermindert werden soll.

Ebenfalls ist die Ausführung dieses Fermentationsverfahrens und der damit zusammenhängenden Prozesse auch in einem diskontinuierlichen Betrieb, beispielsweise "Batch"-Fermentierung denkbar. So kann dem Gärsubstrat gemäß einer weiteren Ausführungsform während der Fermentierung der Mikroorganismus der Art *Clostridium sartagoformum* beispielsweise in regelmäßigen Abständen zugegeben werden. Die Zugabe des Mikroorganismus der Art *Clostridium sartagoformum* in regelmäßigen Abständen führt zu einer Steigerung der Lebendzellzahl und somit zu einem verbesserten Ablauf der fermentativen Prozesse, beispielsweise der Hydrolyse bei einer gleichzeitig verbesserten Ausnutzung des Gärsubstrats für die Fermentierung.

Gemäß einer bevorzugten Ausführungsform der vorliegenden Erfindung wird der Mikroorganismus der Art *Clostridium sartagoformum* in einer Menge zu dem Gärsubstrat zugegeben, so dass nach Zugabe der Anteil des Mikroorganismus der Art *Closfridium sartagoformum* zwischen 10⁻⁴% und 10% der Gesamtzahl an in dem Gärsubstrat anwesenden Mikroorganismen ausmacht. Insbesondere in Abhängigkeit von der Fermentergröße und damit in Abhängigkeit von der Menge an Gärsubstrat kann zur Erzielung der gewünschten Wirkung eine Zugabe von sehr stark unterschiedlichen Mengen an Mikroorganismen notwendig werden.

Sowohl die Bestimmung der Gesamtzahl von Mikroorganismen in dem Gärsubstrat wie auch die Bestimmung der Anteile verschiedener Arten von Mikroorganismen im Gärsubstrat stellt für den Fachmann kein Problem dar. So kann beispielsweise mit Hilfe von Fluoreszenz-markierten Oligosonden spezifisch der Anteil verschiedener Mikroorganismen in dem Gärsubstrat identifiziert werden

Besonders bevorzugt wird der Mikroorganismus der Art *Clostridium sartagoformum* in einer Menge zu dem Gärsubstrat zugegeben, dass nach Zugabe der Anteil des Mikroorganismus der Art *Clostridium sartagoformum* zwischen 10⁻³% und 1 % der Gesamtzahl an in dem Gärsubstrat anwesenden Mikroorganismen ausmacht.

Es soll nochmals darauf hingewiesen werden, dass der Zusatz von Mikroorganismen der Art *Clostridium sartagoformum* zu jedem beliebigen Zeitpunkt des Fermentationsprozesses erfolgen kann, insbesondere können Mikroorganismen der Art *Clostridium sartagoformum* auch zum Animpfen von Gärsubstrats bei der erstmaligen Inbetriebnahme oder einer Wiederinbetriebnahme eines Fermenters verwendet werden.

Ebenso ist es möglich Mikroorganismen der Art *Clostridium sartagoformum* bei Störungen des Fermentationsprozesses zur Stabilisierung der Fermentation zuzugeben. Solche Störungen können durch Überwachung bestimmter charakteristischer Parameter der Fermentierung frühzeitig erkannt werden. Charakteristische Parameter geben Auskunft über die Qualität eines ablaufenden Fermentierungsprozesses zur Herstellung von Biogas. Solche charakteristischen Parameter sind nicht nur die Menge an erzeugtem Biogas und der Methangehalt des erzeugten Biogases sondern beispielsweise auch der Wasserstoffgehalt des erzeugten Biogases, der pH-Wert des Gärsubstrats, das Redoxpotential des Gärsubstrats, der Carbonsäuregehalt des Gärsubstrats, die Anteile verschiedener Carbonsäuren im Gärsubstrat, der Wasserstoffgehalt des Gärsubstrats, der Anteil der Trockensubstanz am Gärsubstrat, der Anteil der organischen Trockensubstanz am Gärsubstrat, die Viskosität des Gärsubstrats und die Raumbelastung des Fermentierungsreaktors.

Ebenso umfasst die vorliegende Erfindung den Stamm des Mikroorganismus *Clostridium sartagoformum SBG1,* wie er unter der Nr. DSM 19861 hinterlegt ist. Der Mikroorganismus *Clostridium sartagoformum SBG1* wurde in einer Reinkultur bei der Deutschen Sammlung von Mikroorganismen und Zellkulturen GmbH in Braunschweig gemäß des Budapester Vertrages hinterlegt. Die Bezeichnung lautet: *Clostridium sartagoformum SBG1* mit der Hinterlegungsnummer DSM 19861.

Bakterien der Art *Clostridium sartagoformum* können mit Hilfe von dem Fachmann bekannten Methoden aus dem Gärsubstrat eines Fermenters isoliert werden. Dabei wird ein geeignetes Substrat aus einem Fermenter in ein Selektionsmedium eingebracht, über längere Zeit kultiviert und schließlich einzelne Kolonien von Mikroorganismen aus dem Selektionsmedium isoliert. Nach Vervielfältigung der daraus erhaltenen mikrobiellen DNA mittels PCR können auf der Basis der 16S rRNA Mikroorganismen der Art *Clostridium sartagoformum* ausgewählt werden.

Bakterien *Clostridium sartagoformum SBG1* wurden aus dem Gärsubstrat eines Nachgärers isoliert. Dazu wurde durch ein flüssiges Selektionsmedium Stickstoff und Kohlendioxid durchgeleitet, anschließend Na₂S zu dem Selektionsmedium zugegeben und autoklaviert (20 Min. bei 121°C). Dann wurde die aus dem Nachgärer gewonnene Biomasse in das Selektionsmedium eingebracht und für zumindest eine Woche bei einer Temperatur von mindestes 30°C kultiviert. Eine aus dem flüssigen Selektionsmedium gewonnen Probe wurde auf ein festes Selektionsmedium aufgebracht und nachfolgend die auf dem festen Selektionsmedium gewachsenen Kolonien von Mikororganismen ausgewählt. Nach Vervielfältigung der erhaltenen mikrobiellen DNA mittels PCR konnte ein Vergleich mit bekannten DNA-Sequenz durchgeführt werden.

Nachdem die Bakterien *Clostridium sartagoformum SBG1* erfolgreich aus dem Gärsubstrat des Nachgärers isoliert worden waren, wurden diese Mikroorganismen einer Sequenzanalyse unterzogen. Die 16S rRNA-Sequenz SEQ ID Nr. 1 umfasst 948 Nukleotide. Als nächster Verwandter wurde ein nicht kultivierter Bakterien Klon aaa62b07 mit der Gensequenz SEQ ID Nr. 2 identifiziert. Ein Vergleich der Sequenzen ergibt, dass insgesamt 9 Austausche von Nukleotiden oder Lücken vorliegen. Bei einer Länge der Sequenz von *Clostridium sartagoformum SBG1* von 948 Nukleotiden errechnet sich mit Hilfe des FASTA-Algorithmus eine Übereinstimmung von 99,2%.

Die vorliegende Erfindung umfasst auch Mikroorganismen mit einer Nukleinsäure, die eine Nukleotidsequenz aufweist, die einen Sequenzbereich enthält, der mehr als 99,2% Sequenzidentität mit der Nukleotidsequenz SEQ ID Nr. 1 aufweist.

Umfasst sind also auch alle Mikroorganismen mit einer Nukleinsäure, deren Nukleotidsequenz zumindest einen Sequenzbereich aufweist, der gegenüber der Nukleotidsequenz SEQ ID Nr. 1 lediglich an einer Position einen Nukleotidaustausch aufweist. Ebenso umfasst sind alle Mikroorganismen, deren DNA-Sequenz zumindest einen Sequenzbereich aufweist, der gegenüber der Nukleotidsequenz SEQ ID Nr. 1 lediglich an zwei Positionen einen Nukleotidaustausch aufweist. Daneben sind alle Mikroorganismen umfasst, deren DNA-Sequenz zumindest einen Sequenzbereich aufweist, der gegenüber der Nukleotidsequenz SEQ ID Nr. 1 lediglich an drei Positionen einen Nukleotidaustausch aufweist.

Außerdem sind auch alle Mikroorganismen, deren DNA-Sequenz zumindest einen Sequenzbereich aufweist, der gegenüber der Nukleotidsequenz SEQ ID Nr. 1 Nukleotidaustausche an vier Positionen aufweist, umfasst. Ebenso umfasst sind alle Mikroorganismen, deren DNA-Sequenz zumindest einen Sequenzbereich aufweist, der gegenüber der Nukleotidsequenz SEQ ID Nr. 1 an fünf Positionen Nukleotidaustausche aufweist. Daneben sind alle Mikroorganismen umfasst, deren DNA-Sequenz zumindest einen Sequenzbereich aufweist, der gegenüber der Nukleotidsequenz SEQ ID Nr. 1 an sechs Positionen Nukleotidaustausche aufweist.

Daneben sind auch alle Mikroorganismen, deren DNA-Sequenz zumindest einen Sequenzbereich aufweist, der gegenüber der Nukleotidsequenz SEQ ID Nr. 1 Nukleotidaustausche an sieben Positionen aufweist, umfasst.

Es versteht sich von selbst, dass die Austausche der Nukleotide an beliebigen Position der DNA-Sequenz vorliegen können. Insbesondere können die Austausche an beliebig weit voneinander entfernten Positionen vorliegen. Sind im Vergleich zur Nukleotidsequenz SEQ ID Nr. 1 beispielsweise sechs Nukleotide ausgetauscht, so können diese sechs ausgetauschten Nukleotide benachbart zueinander vorliegen. In diesem Fall wäre ein sechs Nukleotide langes Teilstück der Nukleotidsequenz SEQ ID Nr. 1 verändert. Ebenso können die sechs ausgetauschten Nukleotide aber beispielsweise jeweils 100 Nukleotide weit voneinander entfernt sein. Die Austausche können also in beliebigen Kombinationen vorliegen.

Ebenso können die genannten Austausche auch in Form von Lücken vorliegen. Umfasst sind also auch alle Mikroorganismen mit einer Nukleinsäure, deren Nukleotidsequenz zumindest einen Sequenzbereich aufweist, in der gegenüber der Nukleotidsequenz SEQ ID Nr. 1 lediglich an einer Position ein Nukleotid fehlt. Ebenso umfasst sind alle Mikroorganismen, deren DNA-Sequenz zumindest einen Sequenzbereich aufweist, in der gegenüber der Nukleotidsequenz SEQ ID Nr. 1 lediglich an zwei Positionen Nukleotide fehlen. Daneben sind alle Mikroorganismen umfasst, deren DNA-Sequenz zumindest einen Sequenzbereich aufweist, in der gegenüber der Nukleotidsequenz SEQ ID Nr. 1 lediglich an drei Positionen Nukleotide fehlen.

Außerdem sind auch alle Mikroorganismen, deren DNA-Sequenz zumindest einen Sequenzbereich aufweist, in dem gegenüber der Nukleotidsequenz SEQ ID Nr. 1 Nukleotide an vier Positionen fehlen, umfasst. Ebenso umfasst sind alle Mikroorganismen, deren DNA-Sequenz zumindest einen Sequenzbereich aufweist, in dem im Vergleich zu der Nukleotidsequenz SEQ ID Nr. 1 an fünf Positionen Nukleotide fehlen. Daneben sind alle Mikroorganismen umfasst, deren DNA-Sequenz zumindest einen Sequenzbereich aufweist, in dem gegenüber der Nukleotidsequenz SEQ ID Nr. 1 an sechs Positionen Nukleotide fehlen.

Daneben sind auch alle Mikroorganismen, deren DNA-Sequenz zumindest einen Sequenzbereich aufweist, in dem gegenüber der Nukleotidsequenz SEQ ID Nr. 1 Nukleotide an sieben Positionen fehlen, umfasst.

Es versteht sich von selbst, dass die Nukleotide an beliebigen Position der DNA-Sequenz fehlen können. Insbesondere können die Lücken an beliebig weit voneinander entfernten Positionen vorliegen. Fehlen im Vergleich zur Nukleotidsequenz SEQ ID Nr. 1 beispielsweise sechs Nukleotide, so können diese sechs fehlenden Nukleotide in der Nukleotidsequenz SEQ ID Nr. 1 benachbart zueinander vorliegen. In diesem Fall würde ein sechs Nukleotide langes Teilstück der Nukleotidsequenz SEQ ID Nr. 1 fehlen. Ebenso können die sechs fehlenden Nukleotide in der Nukleotidsequenz SEQ ID Nr. 1 aber beispielsweise jeweils 100 Nukleotide weit voneinander entfernt sein. Die Lücken können also in beliebigen Kombinationen vorliegen.

Die vorliegende Erfindung umfasst auch eine Kultur von Mikroorganismen geeignet zum Einsatz in einem Verfahren zur fermentativen Erzeugung von Biogas aus Biomasse, wobei in der Kultur von Mikroorganismen ein Mikroorganismus *Clostridium sartagoformum SBG1* anwesend ist wie er unter der Nr. DSM 19861 hinterlegt wurde, wobei der Mikroorganismus zumindest 1% der Gesamtzahl an in der Kultur vorhandenen Mikroorganismen ausmacht.

Die vorliegende Erfindung umfasst auch eine Kultur von Mikroorganismen geeignet zum Einsatz in einem Verfahren zur fermentativen Erzeugung von Biogas aus Biomasse, wobei in der Kultur von Mikroorganismen ein Mikroorganismus anwesend ist, der eine Nukleotidsequenz aufweist, die einen Sequenzbereich enthält, der zumindest 99,2% Sequenzidentität mit der Nukleotidsequenz SEQ ID Nr. 1 aufweist, wobei der Mikroorganismus zumindest 1% der Gesamtzahl an in der Kultur vorhandenen Mikroorganismen ausmacht.

Besonders bevorzugt macht der Mikroorganismus *Clostridium sartagoformum* zumindest 10%, insbesondere bevorzugt zumindest 50% der Gesamtzahl an in der Kultur vorhandenen Mikroorganismen aus.

Besonders bevorzugt handelt es sich um eine Reinkultur von Mikroorganismen geeignet zum Einsatz in einem Verfahren zur fermentativen Erzeugung von Biogas aus Biomasse, wobei es sich um eine Reinkultur des Mikroorganismus *Clostridium sartagoformum SBG1* wie er unter der Nr. DSM 19861 hinterlegt wurde oder wie er oben in Bezug auf seine Nukleotidsequenz charakterisiert wurde, handelt.

Besonders bevorzugt handelt es sich in den oben beschriebenen Fällen um eine immobilisierte Kultur von Mikroorganismen.

### Kurze Beschreibung der Zeichnungen

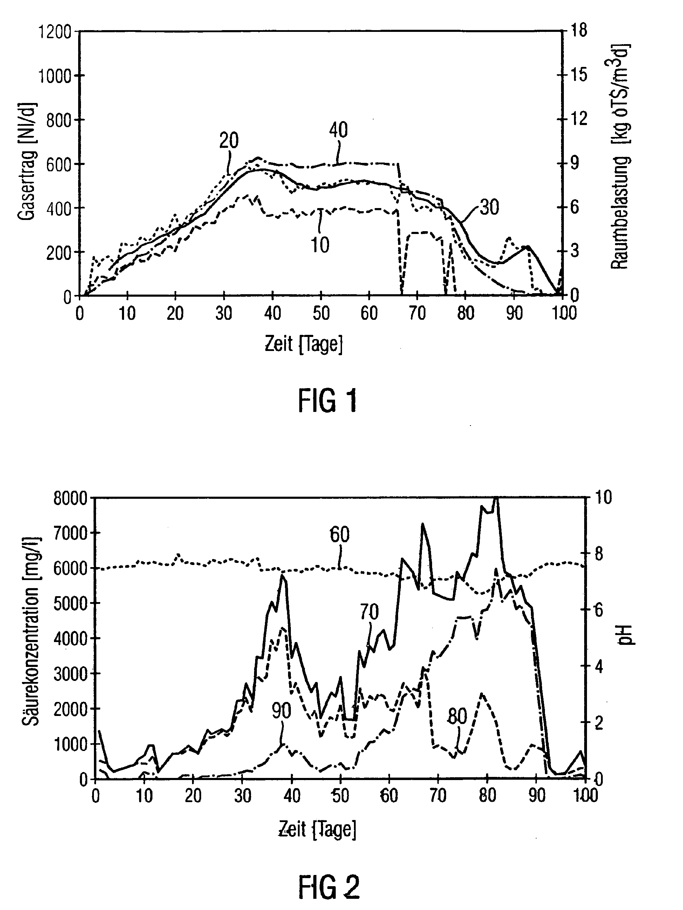

Zur Illustration der Erfindung und zur Verdeutlichung ihrer Vorzüge werden nachfolgend Ausführungsbeispiele angegeben. Die Ausführungsbeispiele sollen im Zusammenhang mit den Figuren 1 bis 6 näher erläutert werden. Es versteht sich von selbst, dass die im Zusammenhang mit den Ausführungsbeispielen gemachten Angaben die Erfindung nicht beschränken sollen. Es zeigen:
- Fig. 1: Messergebnisse einer Fermentierung: Aufgetragen sind die Menge an erzeugtem Biogas, der 6-tägige gleitende Durchschnitt der Menge an erzeugtem Biogas, die theoretische Gasproduktion sowie die Raumbelastung des Fermenters gegen die Zeit;
- Fig. 2: Messergebnisse der Fermentierung gemäß Figur 1: Aufgetragen sind die Konzentration an Essigsäure, die Konzentration an Propionsäure, das Essigsäureäquivalent und der pH-Wert gegen die Zeit;
- Fig. 3: Messergebnisse der Fermentierung gemäß Figur 1: Aufgetragen sind die Raumbelastung des Fermenters, der prozentuale Trockensubstanzanteil im Gärsubstrat und der prozentuale Anteil der organischen Trockensubstanz gegen die Zeit;
- Fig. 4: Messergebnisse einer Fermentierung unter Zugabe von *Clostridium sartagoformum SBG1* und ansonsten identischen Bedingungen wie in der Fermentierung gemäß den Figuren 1 bis 3: Aufgetragen sind die Menge an erzeugtem Biogas, der 6-tägige gleitende Durchschnitt der Menge an erzeugtem Biogas, die theoretische Gasproduktion sowie die Raumbelastung des Fermenters gegen die Zeit;
- Fig. 5: Messergebnisse der Fermentierung gemäß Figur 4: Aufgetragen sind die Konzentration an Essigsäure, die Konzentration an Propionsäure, das Essigsäureäquivalent und der pH-Wert gegen die Zeit;
- Fig. 6: Messergebnisse der Fermentierung gemäß Figur 4: Aufgetragen sind die Raumbelastung des Fermenters, der prozentuale Trockensubstanzanteil im Gärsubstrat und der prozentuale Anteil der organischen Trockensubstanz gegen die Zeit.

### Wege zur Ausführung der Erfindung

### Vergleichsbeispiel

Die Figur 1 zeigt Messergebnisse verschiedener charakteristischer Parameter während eines Fermentationsprozesses in einem Versuchsfermenter mit einem Volumen von 150 l unter realistischen Anlagenbedingungen. Die mit dem Bezugszeichen 10 versehene Kurve zeigt den zeitlichen Verlauf der Raumbelastung des Fermenters in Kilogramm organischer Trockensubstanz je Kubikmeter je Tag (kg oTS/m³d) und die mit dem Bezugszeichen 20 gekennzeichnete Kurve den zeitlichen Verlauf des gesamten erzeugten Biogases in Normliter (Gasvolumen bei 273,15 K und 1013 mbar) je Tag (NI/d). Mit dem Bezugszeichen 30 ist der zeitliche Verlauf des 6-tägigen gleitenden Durchschnitts des gesamt erzeugten Biogases in [NI/d] gekennzeichnet und der zeitliche Verlauf der theoretischen Gasproduktion in [NI/d] ist durch das Bezugszeichen 40 markiert.

Das Kuratorium für Technik und Bauwesen in der Landwirtschaft (KTBL) aber auch die Landesanstalt für Landwirtschaft haben Richtwerte herausgegeben, die in etwa angeben, welche Mengen an Biogas in Abhängigkeit vom eingesetzten Substrat bei einer stabilen Fermentierung zu erwarten sind. Diese theoretischen Richtwerte können somit die Menge an theoretisch erzeugbarem Biogas widerspiegeln. Alternativ können auch von anderen Instituten in anderen Ländern herausgegebene Richtwerte verwendet werden. Der zeitliche Verlauf der theoretischen Gasproduktion 40 in [NI/d] wurde nach solchen Richtwerten berechnet.

Der Figur 1 ist zu entnehmen, dass im Verlauf der ersten 35 Tage ein kontinuierlicher und stabiler Fermentationsprozess stattfindet, der durch einen Anstieg der Raumbelastung auf etwa 6,5 kg oTS/m³d sowie durch eine mit der Raumbelastung kontinuierliche zunehmende Bildung von Biogas gekennzeichnet ist. Ab Tag 36 ist eine Abweichung der erzeugten Biogasmenge von der theoretischen Gasproduktion zu beobachten. Aufgrund einer unkontrolliert hohen Säurekonzentration im Gärsubstrat, die in Figur 2 dargestellt wird, wird die Raumbelastung durch ein Aussetzen der Steigerung der Substratzufuhr ab dem Tag 38 ungefähr konstant gehalten. Da die Säurekonzentrationen jedoch weiterhin unkontrollierbar bleiben, wird am Tag 67 und am Tag 76 kein Substrat zugeführt und die Substratzufuhr ab dem Tag 78 vollständig ausgesetzt. Es zeigte sich, dass eine maximale Raumbelastung von 5,5 kg oTS/m^{s}d erreicht werden konnte. Bei einer höheren Raumbelastung ist aufgrund des Zusammenbruchs des Fermentationsprozesses kein stabiler Betrieb mehr möglich.

Figur 2 zeigt die zeitabhängige Entwicklung der Konzentrationen charakteristischer Carbonsäuren während des bereits im Zusammenhang mit Figur 1 erläuterten Fermentationsprozesses. Gezeigt ist der zeitliche Verlauf des pH-Werts (mit 60 bezeichnete Kurve), des Essigsäureäquivalents zur Bestimmung der flüchtigen Fettsäuren (mit 70 bezeichnete Kurve), der Essigsäurekonzentration (mit 80 bezeichnete Kurve) und der Propionsäurekonzentration (mit 90 bezeichnete Kurve). Die Säurekonzentrationen werden dabei in Milligramm je Liter Gärsubstrat (mg/l) angegeben.

Die Bestimmung flüchtiger Fettsäuren als Summenparameter im Gärsubstrat erfolgte durch Wasserdampfdestillation einer mit Phosphorsäure angesäuerten Gärsubstratprobe. Das Destillat wurde anschließend mit Natronlauge gegen Phenolphthalein titriert. Alternativ bzw. zur Unterscheidung der einzelnen Carbonsäuren ist auch eine gaschromatografische Bestimmung möglich.

Es zeigte sich, dass der pH-Wert in den ersten 35 Tagen des Versuchs nahezu stabil bei pH 7,5 lag. Aufgrund der unkontrolliert hohen Säurekonzentration ab dem Tag 36 wurde, wie im Zusammenhang mit der Figur 1 erläutert, die Substratzufuhr zunächst nicht weiter gesteigert und am Tag 67, am Tag 76 bzw. ab dem Tag 78 vollständig ausgesetzt. Begleitend zur unkontrolliert hohen Säurekonzentration sinkt der pH-Wert leicht in ein schwach saures Milieu ab, womit sich ein instabiler Fermentationsprozess abzuzeichnen beginnt. Der pH-Wert pendelt sich erst dann wieder im neutralen bis leicht alkalischen Milieu ein, wenn die Säurekonzentration gegen Null absinkt. Diese Erholung des pH-Werts ist durch den mikrobiellen Abbau der Säuren im Gärsubstrat zu erklären, unterstützt durch das Fehlen weiterer Substratzufuhr.

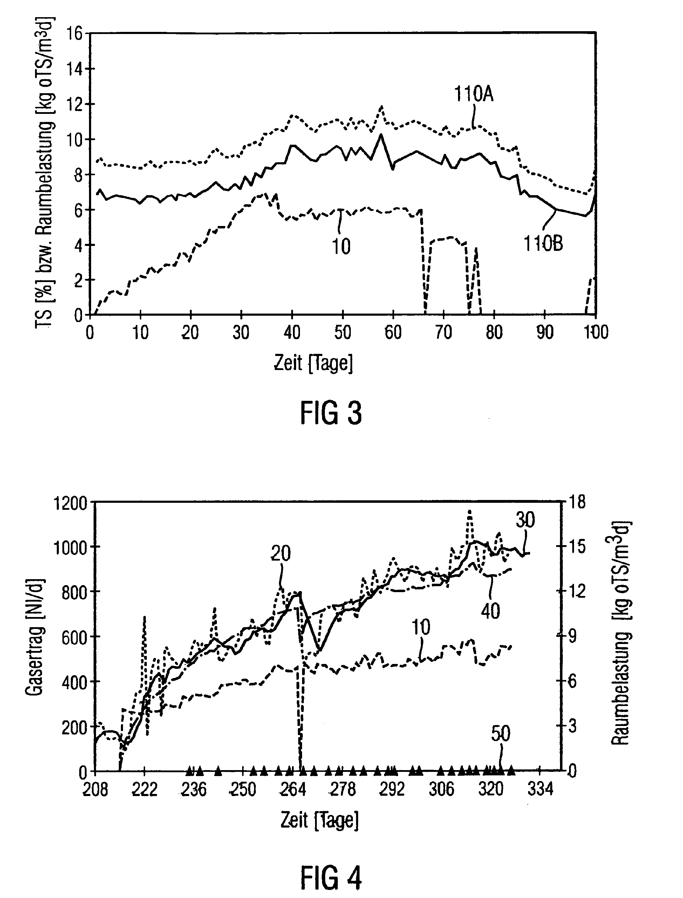

Figur 3 zeigt den Trockensubstanzgehalt des Fermenterinhalts in Abhängigkeit von der Raumbelastung während des bereits im Zusammenhang mit den Figuren 1 und 2 erläuterten Fermentationsprozesses. Gezeigt ist der zeitliche Verlauf der Raumbelastung in [kg oTS/m³d] (mit dem Bezugszeichen 10 gekennzeichnete Kurve), des prozentualen Trockensubstanzanteils (mit dem Bezugszeichen 110A gekennzeichnete Kurve) und des prozentualen Anteils der organischen Trockensubstanz (mit dem Bezugszeichen 110B gekennzeichnete Kurve). Der prozentuale Trockensubstanzanteil gibt die gesamte Masse organischer und anorganischer Substanzen wie beispielsweise Sand an.

Aus Figur 3 lässt sich erkennen, dass mit dem in Figur 2 dargestellten Anstieg der Säurekonzentration am Tag 36 ebenso der prozentuale Trockensubstanzgehalt sowie der prozentuale Gehalt an organischer Trockensubstanz ansteigt. Trotz der vorgenommenen Reduzierung der Raumbelastung am Tag 38, die zwischen den Tagen 38 bis 67 nahezu konstant bei 5,5 bis 6,0 kg oTS/m³d bleibt, reichert sich organische Trockensubstanz 110B an, die nicht mehr ausreichend fermentiert werden kann. Erst mit dem Aussetzen der Substratzufuhr ab Tag 78 erholen sich die biologischen Prozesse, so dass die organische Trockensubstanz wieder fermentiert wird, was an dem Absinken des Anteils der Trockensubstanz 110B ersichtlich ist.

### Closfridium sartagoformum SBG1

Bakterien der Art *Clostridium sartagoformum SBG1* wurden erfolgreich aus dem Gärsubstrat eines Nachgärers isoliert. Die Hinterlegung des Organismus erfolgte in einer Reinkultur bei der Deutschen Sammlung von Mikroorganismen und Zellkulturen GmbH gemäß des Budapester Vertrages *(Clostridium sartagoformum SBG1* mit der Hinterlegungsnummer DSM 19861).

Die Isolation der Mikroorganismen erfolgte mithilfe eines Selektionsmediums, das Carboxymethylcellulose als einzige Kohlenstoffquelle beinhaltete. Carboxymethylcellulose besitzt sehr große Ähnlichkeit mit der in Gärsubstraten von Biogasanlagen enthaltenen Cellulose und weist zudem durch die Verknüpfung der Hydroxylgruppen mit Carboxymethylgruppen (-CH₂-COOH-) eine verbesserte Löslichkeit in wässrigem Medium auf. Das zur Selektion von *Clostridium sartagoformum SBG1* eingesetzte Medium wurde mit N₂ und CO₂ begast, damit die Selektion unter anaeroben Bedingungen erfolgen konnte. Enthaltener Restsauerstoff wurde anschließend mit Hilfe von 0,5 g/l Na₂S reduziert.

Das Selektionsmedium wurde anschließend mit dem Überstand von Material aus einem Nachgärer beimpft. Nach einer einwöchigen Kultivierung bei 40°C zeigten sich bei mikroskopischer Analyse einzelne Stäbchen. Eine weitere Selektion der Flüssigkulturen erfolgte durch den Ausstrich auf anaeroben Carboxymethylcellulose-Platten. Das Zellmaterial der gewachsenen Kolonien diente zur Vervielfältigung der mikrobiellen DNA mittels der Colony-PCR Methode nach einem StandardProgramm. Nach der Sequenzanalyse der Kolonien erbrachte der phylogenetische Vergleich der daraus generierten Sequenzdaten mittels BLAST-Programm (basic local alignment search tool) der Datenbank www.ncbi.nlm.nih.gov, dass die erhaltene Sequenz dem Organismus *Clostridium sartagoformum* als nächstem Verwandten zugeordnet werden kann.

Versuche mit einer Reinkultur des hydrolytisch aktiven, fermentativen Mikroorganismus *Clostridium sartagoformum SBG1* zeigten, dass der Zusatz zum stark zähflüssigen, viskosen Carboxymethylcellulose-haltigen Selektionsmedium zu einer sukzessiven Verflüssigung des Mediums führt.

### Beispiel

Die Figur 4 zeigt Messergebnisse verschiedener charakteristischer Parameter während eines Fermentationsprozesses in einer Versuchsanlage unter den im Zusammenhang mit den Figuren 1 bis 3 beschriebenen Anlagenbedingungen. Die mit dem Bezugszeichen 10 versehene Kurve zeigt den zeitlichen Verlauf der Raumbelastung des Fermenters in Kilogramm organischer Trockensubstanz je Kubikmeter je Tag (kg oTS/m³d) und die mit dem Bezugszeichen 20 gekennzeichnete Kurve den zeitlichen Verlauf des gesamten erzeugten Biogases in Normliter (Gasvolumen bei 273,15 K und 1013 mbar) je Tag (NI/d). Mit dem Bezugszeichen 30 ist der zeitliche Verlauf des 6-tägigen gleitenden Durchschnitts des gesamt erzeugten Biogases in [NI/d] gekennzeichnet und der zeitliche Verlauf der theoretischen Gasproduktion in [NI/d] ist durch das Bezugszeichen 40 markiert.

Im Unterschied zu der im Zusammenhang mit den Figuren 1 bis 3 beschriebenen Fermentierung wurde bei dem nachfolgend mit Bezug auf die Figuren 4 bis 6 diskutierten Fermentationsprozess erfindungsgemäß der Mikroorganismus *Clostridium sartagoformum* dem Gärsubstrat zugesetzt. Die Zeitpunkte der Zugabe von *Clostridium sartagoformum SBG1* sind durch mit dem Bezugszeichen 50 markierte Dreiecke auf der X-Achse gekennzeichnet.

In dem beschriebenen Ausführungsbeispiel wurde eine Reinkultur von *Clostridium sartagoformum* eingesetzt. Ebenso können aber auch Mischkulturen mit einem Anteil an *Clostridium sartagoformum* verwendet werden.

Ab dem Tag 215 wurde eine kontinuierliche Steigerung der Raumbelastung von ca. 4,25 kg oTS/m³d auf ca. 8,5 kg oTS/m³d am Tag 314 vorgenommen. Innerhalb dieses Zeitraums wurde mehrfach eine Reinkultur von *Clostridium sartagoformum SBG1* zum Gärsubstrat hinzugegeben. Der Einbruch der Raumbelastung am Tag 266 ist auf ein technisches Problem zurückzuführen, das an diesem Tag zu einem Ausfall der Substratzufuhr in der Biogasanlage geführt hat.

Der erste Einsatz einer Reinkultur von *Clostridium sartagoformum SBG1* erfolgte am Versuchstag 235. Zum Zeitpunkt der ersten Mikroorganismenzugabe wurde der Fermenter mit einer Raumbelastung von etwa 4,5 kg organischer Trockensubstanz/m³ und Tag betrieben. Dazu wurde die Zellmasse aus 1l einer Vorkultur von *Clostridium sartagoformum SBG1,* die 5 Tage bei einer Temperatur von etwa 40°C inkubiert worden war, eingesetzt. Die Zellzahl dieser Vorkultur besaß eine Zelldichte von ca. 2,0 x 10⁸ Zellen/ml mit einem Anteil lebender Zellen von über 90%. Während die Zugabe zunächst im 1l-Maßstab zweimal wöchentlich erfolgte, wurde die Zugabemenge ab dem Versuchstag 277 verdoppelt, sodass zweimal wöchentlich eine Zugabe der Zellmasse aus 2l einer Vorkultur von *Clostridium sartagoformum SBG1* erfolgte.

Ab Versuchstag 293 wurde dreimal wöchentlich Zellmasse aus 2l einer Vorkultur von *Clostridium sartagoformum SBG1* zugegeben. Zudem wurde die Inkubationsdauer der Vorkultur auf mindestens 7 Tage ausgedehnt, wodurch die Zelldichte auf durchschnittlich 1 x 10⁹ Zellen/ml anstieg. Durch die Zugabe der Reinkulturen von *Clostridium sartagoformum* SGB1 konnte die Raumbelastung im weiteren Versuchsverlauf bis auf einen am Tag 314 erreichten Maximalwert von etwa 8,5 kg oTS/m³d gesteigert werden.

Parallel zur steigenden Raumbelastung konnte eine Steigerung des Biogasertrages beobachtet werden. Dabei war eine Übereinstimmung der erzeugten Biogasmenge in Normliter/Tag [NI/d] mit der theoretischen Gasproduktion in Normliter/Tag [NI/d] zu beobachten, wobei die erzeugte Biogasmenge etwa ab dem Tag 288 nahezu konstant über dem theoretisch zu erwartenden Biogasertrag lag. Dies ist auf die gesteigerte Zugabe der *Clostridium sartagoformum* SBG1-Reinkultur ab dem Tag 277 zurückzuführen.

Nach den Versuchstagen 290 und 308 ist zudem zu beobachten, dass der Gasertrag kurzzeitig deutlich über den theoretischen Wert anstieg, um sich anschließend der theoretischen Kurve wieder zu nähern. Diese Erhöhungen der Biogasproduktion gehen einher mit einer jeweiligen Erhöhung der zugegebenen Mikroorganismenmenge. Daraus lässt sich schließen, dass nach einer Adaptionsphase, in der eine Grundkonzentration an Mikroorganismen aufgebaut werden muss, eine Erhöhung der Zellzahl zu einer Beschleunigung der Hydrolyse führt.

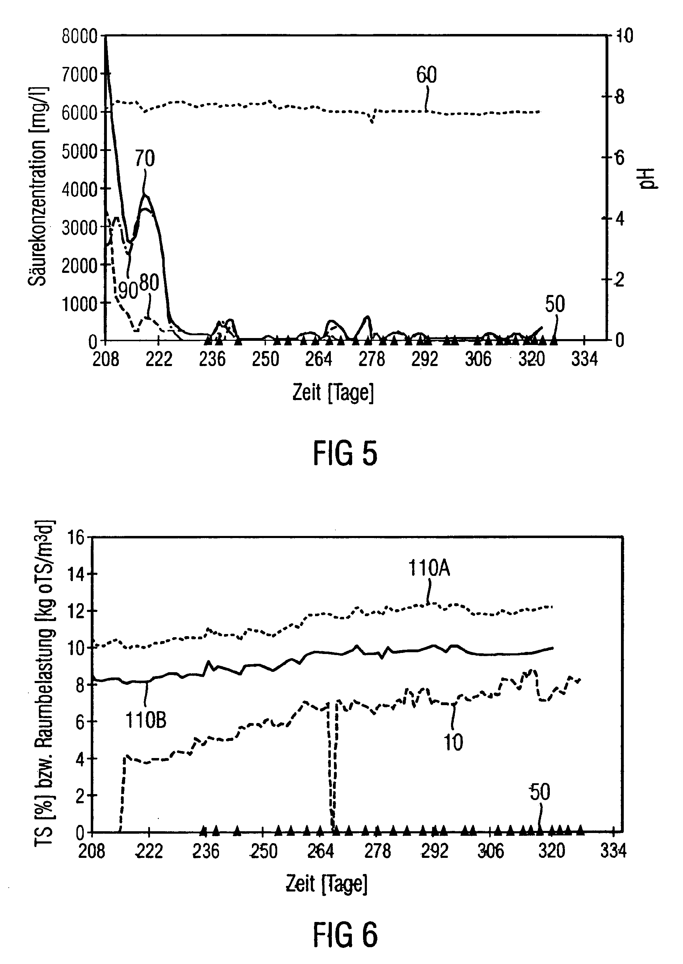

Figur 5 zeigt die zeitabhängige Entwicklung der Konzentrationen charakteristischer Carbonsäuren während des bereits in Zusammenhang mit Figur 4 beschriebenen Fermentationsprozesses. Gezeigt ist der zeitliche Verlauf des pH-Wertes (mit dem Bezugszeichen 60 gekennzeichnete Kurve), des Essigsäureäquivalents zur Bestimmung der flüchtigen Fettsäuren in [mg/l], markiert mit dem Bezugszeichen 70, der Essigsäurekonzentration in [mg/l] (mit dem Bezugszeichen 80 gekennzeichnete Kurve) und der Propionsäurekonzentration in [mg/l] (mit dem Bezugszeichen 90 gekennzeichnete Kurve).

Aus Figur 5 ist zu entnehmen, dass der pH-Wert während des Fermentationsprozesses konstant blieb und sich im neutralen bis leicht alkalischen Milieu zwischen pH 7 und 8 befand. Bereits zu Beginn des Fermentationsprozesses war ein drastischer Abfall der Konzentration des Essigsäureäquivalents, der Essigsäurekonzentration und der Propionsäurekonzentration zu erkennen. Insgesamt blieb die Fettsäurekonzentration dauerhaft niedrig und eine Anreicherung längerkettiger Fettsäuren konnte nicht beobachtet werden.

Weder die im Zusammenhang mit der Figur 4 bereits beschriebene drastisch gesteigerte Substratzufuhr noch die am Tag 266 ausgesetzte Substratzufuhr bewirkte auffällige Spitzen in der Konzentration der Carbonsäuren. Das bedeutet, dass die in der Hydrolyse und in der sich anschließenden Acido- und Acetogenese erzeugten Zwischenprodukte während der Methanogenese als letztem Schritt der Biogassynthese zu Biogas umgesetzt werden. Somit wurde gezeigt, dass die Fermentationsprozesse auch bei lang anhaltender hoher Belastung gut ablaufen, so dass keine Anreicherung von organischen Zwischenprodukten stattfindet und folglich eine Langzeitstabilität des Fermentationsprozesses gegeben ist.

Figur 6 zeigt den Trockensubstanzgehalt des Fermenterinhalts und die Raumbelastung während des bereits im Zusammenhang mit den Figuren 4 und 5 erläuterten Fermentationsprozesses. Gezeigt ist der zeitliche Verlauf der Raumbelastung in [kg oTS/m³d] (mit dem Bezugszeichen 10 gekennzeichnete Kurve), des prozentualen Trockensubstanzanteils (mit dem Bezugszeichen 110A gekennzeichnete Kurve) und des prozentualen Anteils der organischen Trockensubstanz (mit dem Bezugszeichen 110B gekennzeichnete Kurve).

Zum Zeitpunkt der ersten Zugabe einer Reinkultur von *Clostridium sartagoformum SBG1* am Versuchstag 235 wurde der Fermenter mit einer Raumbelastung von etwa 4.5 kg organischer Trockensubstanz/m³ und Tag betrieben. Die Raumbelastung der Anlage konnte infolge der Zugabe von *Clostridium sartagoformum SBG1* weiter gesteigert werden. Dabei blieb der prozentuale Gehalt an Trockensubstanz beziehungsweise organischer Trockensubstanz nahezu konstant. Diese Beobachtung legt nahe, dass während der Fermentierung des Gärsubstrats keine Anhäufung von nicht-fermentierter organischer Trockensubstanz erfolgt. Die Zugabe von Reinkulturen von *Clostridium sartagoformum SBG1* trägt also zu einer kontinuierlichen Umsetzung der enthaltenen Trockenmasse im Gärsubstrat bei, welche wiederum zu einer kontinuierlichen Fermentierung führt, indem die Anhäufung von Trockensubstanz vermindert wird.

In Phasen, in denen die Biogasanlage bei konstanter Raumbelastung betrieben wird, ist sogar eine Abnahme der Trockensubstanz zu beobachten, was darauf schließen lässt, dass die *Clostridium sartagoformum* SBG1-Mikroorganismen mit ihrer hydrolytischen Stoffwechselaktivität nicht nur die Anhäufung von Trockensubstanz im Fermenter vermindern, sondern auch die hydrolytische Umsetzung dieser Trockensubstanz verbessern.

Damit belegen die Figuren 4 bis 6 den positiven Effekt der Zugabe des hydrolytisch aktiven, fermentativen Mikroorganismus *Clostridium sartagoformum SBG1* auf die Hydrolyse organischer Trockensubstanz. Durch die Zugabe von Mikroorganismen der Art *Clostridium sartagoformum* kann die Raumbelastung eines Fermenters unter ansonsten identischen Bedingungen von etwa 5,5 kg oTS/m³d auf rund 8,5 kg oTS/m³d, also um mehr als 50% gesteigert werden, ohne dass sich eine Instabilität des Fermentationsprozesses auch nur andeuten würde. Parallel zu der erhöhten Raumbelastung wird die Menge an gebildetem Biogas mehr als verdoppelt. Zudem steigt die spezifische Ausbeute an Biogas an, da deutlich mehr der organischen Trockensubstanz abgebaut wird als bei fehlender Zugabe von Mikroorganismen der Art *Clostridium sartagoformum.* Der Einsatz von Mikroorganismen der Art *Clostridium sartagoformum* führt zu einer dramatischen Verbesserung von Effizienz und Wirkungsgrad von Biogasanlagen.

## Patentansprüche

1. Verfahren zur Erzeugung von Biogas aus Biomasse in einem Fermentierungsreaktor, **dadurch gekennzeichnet, dass** der Biomasse ein Mikroorganismus der Art *Clostridium sartagoformum* in Form einer Kultur von Mikroorganismen zugesetzt wird, wobei der Mikroorganismus der Art *Clostridium sartagoformum* zumindest 1% der Gesamtzahl an in der Kultur vorhandenen Mikroorganismen ausmacht.

2. Verfahren nach Anspruch 1, **dadurch gekennzeichnet, dass** in der Kultur von Mikroorganismen der Mikroorganismus der Art *Clostridium sartagoformum* zumindest 10% der Gesamtzahl an in der Kultur vorhandenen Mikroorganismen ausmacht.

3. Verfahren nach einem der vorhergehenden Ansprüche, **dadurch gekennzeichnet, dass** eine Reinkultur des Mikroorganismus der Art *Clostridium sartagoformum* zugesetzt wird.

4. Verfahren nach einem der vorhergehenden Ansprüche, **dadurch gekennzeichnet, dass** der Mikroorganismus der Art *Clostridium sartagoformum* der Biomasse als Bestandteil zumindest einer immobilisierten Kultur von Mikroorganismen zugesetzt wird.

5. Verfahren nach einem der vorhergehenden Ansprüche, **dadurch gekennzeichnet, dass** zeitnah zur Zugabe des Mikroorganismus der Art *Clostridium sartagoformum* zusätzliche Biomasse in den Fermentierungsreaktor gegeben wird, wobei die Raumbelastung im Fermentierungsreaktor durch kontinuierliche Zugabe von Biomasse kontinuierlich gesteigert wird.

6. Verfahren nach einem der vorhergehenden Ansprüche, **dadurch gekennzeichnet, dass** die Erzeugung von Biogas aus Biomasse bei einer Raumbelastung von ≥ 0,5 kg oTS/m³d, bevorzugt ≥ 4,0 kg oTS/m³d, besonders bevorzugt ≥ 8,0 kg oTS/m³d, durchgeführt wird.

7. Verfahren nach einem der vorhergehenden Ansprüche, **dadurch gekennzeichnet, dass** der Mikroorganismus der Art *Clostridium sartagoformum* in einer Menge zu dem Gärsubstrat zugegeben wird, dass nach Zugabe der Anteil des Mikroorganismus der Art *Clostridium sartagoformum* zwischen 10⁻⁴% und 10% der Gesamtzahl an in dem Gärsubstrat anwesenden Mikroorganismen ausmacht.

8. Verfahren nach Anspruch 7, **dadurch gekennzeichnet, dass** der Mikroorganismus der Art *Clostridium sartagoformum* in einer Menge zu dem Gärsubstrat zugegeben wird, dass nach Zugabe der Anteil des Mikroorganismus der Art *Clostridium sartagoformum* zwischen 10⁻³% und 1% der Gesamtzahl an in dem Gärsubstrat anwesenden Mikroorganismen ausmacht.

9. Stamm des Mikroorganismus *Clostridium sartagoformum SBG1,* der bei der DSMZ unter der Nr. 19861 hinterlegt ist.

10. Mikroorganismus mit einer Nukleinsäure, die eine Nukleotidsequenz aufweist, **dadurch gekennzeichnet, dass** die Nukleotidsequenz einen Sequenzbereich enthält, der mehr als 99,2% Sequenzidentität mit der Nukleotidsequenz SEQ ID Nr. 1 aufweist.

11. Kultur von Mikroorganismen geeignet zum Einsatz in einem Verfahren zur fermentativen Erzeugung von Biogas aus Biomasse, **dadurch gekennzeichnet, dass** in der Kultur von Mikroorganismen ein Mikroorganismus *Clostridium sartagoformum* gemäß den Ansprüchen 9 oder 10 anwesend ist, wobei der Mikroorganismus *Clostridium sartagoformum* zumindest 1% der Gesamtzahl an in der Kultur vorhandenen Mikroorganismen ausmacht.

12. Kultur von Mikroorganismen nach Anspruch 11, **dadurch gekennzeichnet, dass** der Mikroorganismus *Clostridium sartagoformum* zumindest 10% der Gesamtzahl an in der Kultur vorhandenen Mikroorganismen ausmacht.

13. Kultur von Mikroorganismen nach Anspruch 12, **dadurch gekennzeichnet, dass** der Mikroorganismus *Clostridium sartagoformum* zumindest 50% der Gesamtzahl an in der Kultur vorhandenen Mikroorganismen ausmacht.

14. Kultur von Mikroorganismen nach Anspruch 13, **dadurch gekennzeichnet, dass** es sich um eine Reinkultur des Mikroorganismus *Clostridium sartagoformum* handelt.

15. Kultur von Mikroorganismen nach zumindest einem der Ansprüche 11 bis 14, **dadurch gekennzeichnet, dass** es sich um eine immobilisierte Kultur von Mikroorganismen handelt.

## Claims

1. A process for the generation of biogas from biomass in a fermentation reactor, **characterized in that** a micro-organism of the species *Clostridium sartagoformum* is added to the biomass in the form of a culture of microorganisms, wherein the microorganism of the species *Clostridium sartagoformum* makes up at least 1% of the total number of microorganisms present in the culture.

2. The process as claimed in claim 1, **characterized in that**, in the culture of microorganisms, the microorganism of the species *Clostridium sartagoformum* makes up at least 10% of the total number of microorganisms present in the culture.

3. The process as claimed in one of the previous claims, **characterized in that** a pure culture of the microorganism of the species *Clostridium sartagoformum* is added.

4. The process as claimed in one of the previous claims, **characterized in that** the microorganism of the species *Clostridium sartagoformum* is added to the biomass as a component of at least one immobilized culture of microorganisms.

5. The process as claimed in one of the previous claims, **characterized in that**, close to the time of the addition of the microorganism of the species *Clostridium sartagoformum,* additional biomass is added to the fermentation reactor, whereby the volume loading in the fermentation reactor is continuously increased by continuous addition of biomass.

6. The process as claimed in one of the previous claims, **characterized in that** the generation of biogas from biomass is performed at a volume loading of ≥ 0.5 kg oDS/m³d, preferably ≥ 4.0 kg oDS/m³d, particularly preferably ≥ 8.0 kg oDS/m³d.

7. The process as claimed in one of the previous claims, **characterized in that** the microorganism of the species *Clostridium sartagoformum* is added to the fermentation substrate in a quantity such that, after addition, the content of the microorganism of the species *Clostridium sartagoformum* makes up between 10⁻⁴% and 10% of the total number of microorganisms present in the fermentation substrate.

8. The process as claimed in claim 7, **characterized in that** the microorganism of the species *Clostridium sartagoformum* is added to the fermentation substrate in a quantity such that, after addition, the content of the microorganism of the species *Clostridium sartagoformum* makes up between 10⁻³% and 1% of the total number of microorganisms present in the fermentation substrate.

9. Strain of the microorganism *Clostridium sartagoformum SBG1* which is deposited at the DSMZ under the No. 19861.

10. A microorganism with a nucleic acid, which has a nucleotide sequence, **characterized in that** the nucleotide sequence contains a sequence region which has more than 99.2% sequence identity with the nucleotide sequence SEQ ID No. 1.

11. A culture of microorganisms suitable for use in a process for the fermentative generation of biogas from biomass, **characterized in that**, in the culture of microorganisms, a microorganism *Clostridium sartagoformum* as claimed in claims 9 or 10 is present, wherein the microorganism *Clostridium sartagoformum* makes up at least 1% of the total number of microorganisms present in the culture.

12. The culture of microorganisms as claimed in claim 11, **characterized in that** the microorganism *Clostridium sartagoformum* makes up at least 10% of the total number of microorganisms present in the culture.

13. The culture of microorganisms as claimed in claim 12, **characterized in that** the microorganism *Clostridium sartagoformum* makes up at least 50% of the total number of microorganisms present in the culture.

14. The culture of microorganisms as claimed in claim 13, **characterized in that** it is a pure culture of the microorganism *Clostridium sartagoformum.*

15. The culture of microorganisms as claimed in at least one of claims 11 to 14, **characterized in that** it is an immobilized culture of microorganisms.

## Revendications

1. Procédé de production de biogaz à partir de biomasse dans un réacteur de fermentation, **caractérisé en ce qu'**un microorganisme de l'espèce *Clostridium sartagoformum* est ajouté à la biomasse sous la forme d'une culture de microorganismes, le microorganisme de l'espèce *Clostridium sartagoformum* constituant au moins 1% du nombre total des microorganismes présents dans la culture.

2. Procédé selon la revendication 1, **caractérisé en ce que** le microorganisme de l'espèce *Clostridium sartagoformum* constitue au moins 10% du nombre total des microorganismes présents dans la culture de microorganismes.

3. Procédé selon l'une des revendications précédentes, **caractérisé en ce qu'**un bouillon de culture du microorganisme de l'espèce *Clostridium sartagoformum* est utilisé.

4. Procédé selon l'une des revendications précédentes, **caractérisé en ce que** le microorganisme de l'espèce *Clostridium sartagoformum* est ajouté à la biomasse en tant que constituant d'au moins une culture immobilisée de microorganismes.

5. Procédé selon l'une des revendications précédentes, **caractérisé en ce qu'**au moment de l'ajout du microorganisme de l'espèce *Clostridium sartagoformum,* de la biomasse supplémentaire est ajoutée dans le réacteur de fermentation, l'occupation d'espace dans le réacteur de fermentation étant continuellement augmentée par l'ajout continuel de biomasse.

6. Procédé selon l'une des revendications précédentes, **caractérisé en ce que** la production de biogaz à partir de la biomasse est effectuée avec une occupation d'espace supérieure ou égale à 0,5 kg d'oTS/m3d, de préférence supérieure ou égale à 4,0 kg d'oTS/m3d, de façon particulièrement préférée supérieure ou égale à 8,0 kg d'oTS/m3d.

7. Procédé selon l'une des revendications précédentes, **caractérisé en ce que** le microorganisme de l'espèce *Clostridium sartagoformum* est ajouté au substrat de fermentation en une quantité telle qu'après ajout de la proportion de microorganisme de l'espèce *Clostridium sartagoformum,* il représente entre 10-4% et 10% du nombre total des microorganismes présents dans le substrat de fermentation.

8. Procédé selon la revendication 7, **caractérisé en ce que** le microorganisme de l'espèce *Clostridium sartagoformum* est ajouté au substrat de fermentation en une quantité telle qu'après ajout de la proportion de microorganisme de l'espèce *Clostridium sartagoformum,* il représente entre 10-3% et 1% du nombre total des microorganismes présents dans le substrat de fermentation.

9. Souche du microorganisme *Clostridium sartagoformum* SBG1, déposée auprès de la DSMZ sous le numéro 19861.

10. Microorganisme ayant un acide nucléique présentant une séquence de nucléotides, **caractérisé en ce que** la séquence de nucléotides contient un domaine de séquence qui présente plus de 99,2% d'identité de séquence avec la séquence de nucléotides SEQ ID n°1.

11. Culture de microorganismes appropriée pour une utilisation dans un procédé de production par fermentation de biogaz à partir de biomasse, **caractérisée en ce qu'**un microorganisme *Clostridium sartagoformum* selon la revendication 9 ou 10 est présent dans la culture de microorganismes, le microorganisme *Clostridium sartagoformum* constituant au moins 1% du nombre total des microorganismes présents dans la culture.

12. Culture de microorganismes selon la revendication 11, **caractérisée en ce que** le microorganisme *Clostridium sartagoformum* constitue au moins 10% du nombre total des microorganismes présents dans la culture.

13. Culture de microorganismes selon la revendication 12, **caractérisée en ce que** le microorganisme *Clostridium sartagoformum* constitue au moins 50% du nombre total des microorganismes présents dans la culture.

14. Culture de microorganismes selon la revendication 13, **caractérisée en ce qu'**il s'agit d'un bouillon de culture du microorganisme *Clostridium sartagoformum.*

15. Culture de microorganismes selon l'une au moins des revendications 11 à 14, **caractérisée en ce qu'**il s'agit d'une culture immobilisée de microorganismes.


### SEQUENCE LISTING

<110> Schmack Biogas AG
<120> Clostridium sartagoformum zur Erzeugung von Biogas
<130> SCM054WO2
<150> DE 10 2008 003 805.9
   <151> 2008-01-10
<160> 2
<170> PatentIn version 3.3
<210> 1
   <211> 948
   <212> RNA
   <213> Clostridium Sartagoformum SBG1
<400> 1
   cggcgugccu accaugcaag ucgagcgagg agauucccuu cggggaugaa ccuagcggcg
      60
   gacgggugag uaacacgugg gcaaccugcc uuauagaggg gaauagccuu ccgaaaggaa
      120
   gauuaauacc gcauaacauu acauuuucgc augaagaagu aauuaaagga gcaauccgcu
      180
   auaagauggg cccgcggcgc auuagcuagu uggugaggua acggcucacc aaggcgacga
      240
   ugcguagccg accugagagg gugaucggcc acauugggac ugagacacgg cccagacucc
      300
   uacgggaggc agcagugggg aauauugcac aaugggggaa acccugaugc agcaacgccg
      360
   cgugagugau gaaggucuuc ggaucguaaa gcucugucuu cagggacgau aaugacggua
      420
   ccugaggagg aagccacggc uaacuacgug ccagcagccg cgguaauacg uagguggcga
      480
   gcguuguccg gauuuacugg gcguaaaggg agcguaggcg gauuuuuaag ugagauguga
      540
   aauacccggg cucaacuugg gugcugcauu ucaaacugga agucuagagu gcaggagagg
      600
   agaguggaau uccuagugua gcggugaaau gcguagagau uaggaagaac accaguggcg
      660
   aaggcgacuc ucuggacugu aacugacgcu gaggcucgaa agcgugggga gcaaacagga
      720
   uuagauaccc ugguagucca cgccguagac gaugaauacu agguguaggg guugucauga
      780
   ccucugugcc gccgcagacg cauuaaguau uccgccuggg gaguacgguc gcaagauuaa
      840
   aacucaaagg aauugacggg gcccgcacca gcagcggagc augugguuua auucgaagca
      900
   ccgcgagaac cuuaccuaga cuugacaucu ccugaauuac ucuuaauc
      948
<210> 2
   <211> 1441
   <212> DNA
   <213> nicht kultivierter Bakterien Klon aaa62b07
<400> 2
   gacgaacgct ggcggcgtgc ctaacacatg caagtcgagc gaggagattc ccttcgggga 60
   tgaacctagc ggcggacggg tgagtaacac gtgggcaacc tgccttatag aggggaatag 120
   ccttccgaaa ggaagattaa taccgcataa cattacattt tcgcatgaag aagtaattaa 180
   aggagtaatc cgctataaga tgggcccgcg gcgcattagc tagttggtga ggtaacggct 240
   caccaaggcg acgatgcgta gccgacctga gagggtgatc ggccacattg ggactgagac 300
   acggcccaga ctcctacggg aggcagcagt ggggaatatt gcacaatggg ggaaaccctg 360
   atgcagcaac gccgcgtgag tgatgaaggt cttcggatcg taaagctctg tcttcaggga 420
   cgataatgac ggtacctgag gaggaagcca cggctaacta cgtgccagca gccgcggtaa 480
   tacgtaggtg gcgagcgttg tccggattta ctgggcgtaa agggagcgta ggcggatttt 540
   taagtgagat gtgaaatacc cgggctcaac ttgggtgctg catttcaaac tggaagtcta 600
   gagtgcagga gaggagagtg gaattcctag tgtagcggtg aaatgcgtag agattaggaa 660
   gaacaccagt ggcgaaggcg actctctgga ctgtaactga cgctgaggct cgaaagcgtg 720
   gggagcaaac aggattagat accctggtag tccacgccgt agacgatgaa tactaggtgt 780
   aggggttgtc atgacctctg tgccgccgca aacgcattaa gtattccgcc tggggagtac
   840
   ggtcgcaaga ttaaaactca aaggaattga cgggggcccg cacaagcagc ggagcatgtg
   900
   gtttaattcg aagcaacgcg aagaacctta cctagacttg acatctcctg aattactctt
   960
   aatcgaggaa gtcccttcgg agacaggaag acaggtggtg catggttgtc gtcagctcgt
   1020
   gtcgtgagat gttgggttaa gtcccgcaac gagcgcaacc cttattgtta gttgctacca
   1080
   ttaagttgag cactctagca agactgcccg ggttaaccgg gaggaaggtg gggatgacgt
   1140
   caaatcatca tgccccttat gtctagggct acacacgtgc tacaatggca agtacaaaaa
   1200
   gatgcaatac cgcaaggtgg agctaaactt aaaaacttgt ctcagttcgg attgtaggct
   1260
   gaaactcgcc tacatgaagc tggagttgct agtaatcgcg aatcagaatg tcgcggtgaa
   1320
   tacgttcccg ggccttgtac acaccgcccg tcacaccatg agagttggca atacccaaag
   1380
   ttcgtgagct aacccgtaag ggaggcagcg acctaaggta gggtcagcga ttggggtgaa
   1440
   g
   1441